# EUROPEAN PATENT SPECIFICATION

(11) **EP 1 307 538 B1**
(45) Date of publication and mention of the grant of the patent: **07.01.2009**
(21) Application number: 01963833.7
(22) Date of filing: 07.08.2001
(51) Int. Cl.: C12Q 1/26, C12Q 1/44, C12Q 1/00, G01N 33/53

(54) **DETECTION OF COX-2 ACTIVITY AND ANANDAMIDE METABOLITES**
Nachweis der COX-2 Aktivität und von Anandamid-Metaboliten
DETECTION DE L'ACTIVITE COX-2 ET DES METABOLITES D'ANANDAMIDE

(30) Priority: 07.08.2000 US 223665 P; 03.07.2001 US 302975 P
(43) Date of publication of application: 07.05.2003
(73) Proprietor: VANDERBILT UNIVERSITY, Nashville, TN 37240 (US)
(72) Inventor: MARNETT, Lawrence, J., Nashville, TN 37215 (US); KOZAK, Kevin, R., Nashville, TN 37212 (US)
(74) Representative: Thomas, Simon
(86) International application number: PCT/US2001/024796
(87) International publication number: WO 2002/012445

(56) References cited:
- US-A- 5 475 021
- US-A- 5 543 297
- US-A- 6 045 773
- US-B1- 6 207 700
- YU MING ET AL: "Synthesis of prostaglandin E-2 ethanolamide from anandamide by cyclooxygenase-2" JOURNAL OF BIOLOGICAL CHEMISTRY, vol. 272, no. 34, 1997, pages 21181-21186, XP002308187 ISSN: 0021-9258
- AU: Burstein SUMNER ET AL: "Putative metabolites of anandamide: activities and occurrence" Abstracts/ PROSTAGLANDINS AND OTHER LIPID MEDIATORS, BUTTERWORTH, STONEHAM, MA, US, vol. 59, no. 1-6, (1999-12-00) page 50 ISSN: 0090-6980 XP002308190
- BELVISI MARIA G ET AL: "Induction of cyclo-oxygenase-2 by cytokines in human cultured airway smooth muscle cells: Novel inflammatory role of this cell type" BRITISH JOURNAL OF PHARMACOLOGY, vol. 120, no. 5, 1997, pages 910-916, XP002308188 ISSN: 0007-1188
- CREW TRACEY E ET AL: "A cyclooxygenase-2 (COX-2) selective non-steroidal anti-inflammatory drug enhances the growth inhibitory effect of butyrate in colorectal carcinoma cells expressing COX-2 protein: Regulation of COX-2 by butyrate" CARCINOGENESIS (OXFORD), vol. 21, no. 1, January 2000 (2000-01), pages 69-77, XP002308189 ISSN: 0143-3334
- MITCHELL J A ET AL: "SELECTIVITY OF NONSTEROIDAL ANTIINFLAMMATORY DRUGS AS INHIBITORS OFCONSTITUTIVE AND INDUCIBLE CYCLOOXYGENASE" PROCEEDINGS OF THE NATIONAL ACADEMY OF SCIENCES OF USA, NATIONAL ACADEMY OF SCIENCE. WASHINGTON, US, vol. 90, December 1994 (1994-12), pages 11693-11697, XP002032992 ISSN: 0027-8424
- BARNETT J ET AL: "PURIFICATION, CHARACTERIZATION AND SELECTIVE INHIBITION OF HUMAN PROSTAGLANDIN G/H SYNTHASE 1 AND 2 EXPRESSED IN THE BACULOVIRUS SYSTEM" BBA - PROTEIN STRUCTURE AND MOLECULAR ENZYMOLOGIE, ELSEVIER SCIENCE BV, AMSTERDAM, NL, vol. 1209, no. 1, 1994, pages 130-139, XP008015747 ISSN: 0167-4838
- BURSTEIN S H ET AL: "Oxidative metabolism of anandamide" PROSTAGLANDINS AND OTHER LIPID MEDIATORS, BUTTERWORTH, STONEHAM, MA, US, vol. 61, no. 1-2, April 2000 (2000-04), pages 29-41, XP004197448 ISSN: 0090-6980
- BISHOP-BAILEY DAVID ET AL: "Induction of cyclooxygenase-2 in human saphenous vein and internal mammary artery" ARTERIOSCLEROSIS THROMBOSIS AND VASCULAR BIOLOGY, vol. 17, no. 9, 1997, pages 1644-1648, XP008039588 ISSN: 1079-5642

## Description

### 2.0 TECHNICAL FIELD

The present invention relates generally to the cyclooxygenases and their roles in human pathology, including cancer and inflammation. More particularly, this invention pertains to methods for detecting or measuring COX-2 activity by detecting and measuring COX-2 specific enzymatic products including prostaglandin ethanolamides.

COX is a prostaglandin endoperoxide synthase enzyme (cyclooxygenase, COX, EC 1.14.99.1), which catalyzes the conversion of arachidonic acid to prostaglandin (PG) H₂. Two isoforms of COX are known, COX-1 and COX-2. COX-1 is constitutively expressed. COX-2, however, is inducible in a variety of cells, especially those of the central nervous and immune systems (Masferrer et al. 1994, Proc. Natl. Acad. Sci. USA 91:3228-3232; Vane et al. 1994, Proc. Natl. Acad. Sci. USA 91:2046-2050; Kennedy et al. 1993, Biochem. Biophys. Res. Commun. 197:494-500). Certain changes in COX-2 activity are associated with a variety of human inflammatory diseases. These diseases include, but are not limited to, acute appendicitis, asthma, myocardial infarction, certain immunological disease processes, infection, malignancy, endotoxemia and reperfusion injury. In addition, inappropriate COX-2 expression or over-expression is associated with certain types of cancers, including, but not limited to, carcinoma of the colon, rectum, stomach, esophagus, lung, and skin. The amount of COX-2 expression is related to the stage or progression of cancer (Fosslien, E, et al. 2000, Ann. Clin. Lab. Sci. 30:3-21). COX-2 has become a major pharmaceutical target for developing treatments for these and other diseases. Methods of detecting and measuring COX-2 activity are highly desired.

Yu et al. (1997) J. Biol. Chem. 272:21181-21186, describes the enzymatic conversion of arachidonyl ethanolamide (anandamide, AEA), to PGE₂-ethanolamide in cell lines expressing COX-2 but not COX-1.

U.S. Patent 5,543,297 to Cromlish et al*.*, describes measuring total COX activity (COX-1 activity and COX-2 activity) in separate samples, with and without a COX-2 specific inhibitor, and then indirectly estimating COX-2 specific activity by subtracting the total COX activity observed with the inhibitor from the total COX activity observed without the inhibitor. One major weakness of this method is that the dynamics of enzymatic inhibition change based upon numerous variables including time, temperature, concentration, specificity, sample preparation, etc.

U.S. patent 5,475,021 to Marnett et al*.* describes a method of measuring the activity of purified COX-2 by measuring O₂-uptake during catalysis. This method requires purification of the enzyme.

U.S. Patent 6,045,773 to Isakson et al*.,* describes a method for measuring COX-2 activity in a mammal by administering a positron-emitting radioisotppe-labeled COX-2 selective-binding agent to the mammal and then detecting the label by positron-emission tomography (PET). Weaknesses of this method include the invasive nature and expense of PET equipment. In addition, the method only localizes COX-2 protein but does not detect or measure activity.

What is needed, then, is a less-invasive, direct method of selectively detecting and measuring COX-2 activity in biological samples from animals without the need to purify the enzyme.

The present invention provides, in part, methods for selectively detecting and measuring COX-2 enzymatic activity. One object of the invention is to provide a system for distinguishing between COX-2 activity and COX-1 activity. COX-1 and COX-2 are well known to catalyze the committed step in the conversion of arachidonic acid to PGH₂. Downstream event, both enzymatic and non-enzymatic, further convert the PGH₂ into a variety of prostaglandins and thromboxanes. The present invention provides herein methods to exploit a COX-2 selective enzymatic reaction with AEA for specifically detecting and measuring. COX-2 enzymatic activity.

In certain embodiments of the present invention, COX-2 enzymatic activity is detected or measured by detecting or measuring novel PGH₂-EA metabolites, namely : PGB₂-EA, PGD₂-EA, PGE₂-EA, PGF₂α-EA, TxB₂-EA, 6-keto-PGF₁α-EA, 15-keto-PGE₂-EA, 13,14-dihydro-15-keto-PGE₂-EA, PGG₂-EA, PGH₂-EA, PGA₂-EA, PGJ₂-EA, PGJ₂-EA derivatives, bicyclo-PGE₂-EA, TxA₂-EA and PGI₂-EA. In certain embodiments, the COX-2 specific enzymatic activity is compared to COX-1 activity, for example, a COX-2/COX-1 ratio is reported.

### 3.0 DISCLOSURE OF THE INVENTION

One aspect of the present invention is a method of detecting COX-2 activity in a biological system or subject comprising detecting a PGH₂-EA metabolite in a sample of the system. In certain embodiments, detection or measurement of a PGH₂-EA metabolite is performed wherein the metabolite is PGB₂-EA, PGD₂-EA, PGE₂-EA, PGF₂α-EA, TxB₂-EA, 6-keto-PGF₁α-EA, 15-keto-PGE₂-EA, 13,14-dihydro-15-keto-PGE₂-EA, PGG₂-EA, PGH₂-EA, PGA₂-EA, PGJ₂-EA, PGJ₂-EA derivatives, bicyclo-PGE₂-EA, TxA₂-EA or PGI₂-EA. The presence of the PGH₂-EA metabolite in the sample is indicative of the COX-2 activity in the system or subject.

In certain embodiments of the present disclosure the inventors provide standards for determining a relative or absolute measurement of COX-2 activity by comparison to a standard or a standard curve generated using the standard. For example, a standard can be used to generate a standard curve for normalization of particular test results. The present invention provides standards including compounds related to reaction products derived from COX-2 action on AEA incorporating isotopic (*e.g.*, radioactive), fluorescent, enzymatic labels and the like.

In certain embodiments, a determination of the amount of COX-2 activity, by the measurement of PGH₂-EA metabolites, is used to detect inflammation or cancer. In certain embodiments, a relative or absolute determination of inflammation or cancer in the subject is made.
FIG. 1 is a diagram of the chemical structure for arachidonic acid (AA).
FIG. 2 is a diagram of the COX biosynthetic pathway of arachidonic acid to prostaglandins and thromboxane.
FIG. 3 is a diagram of the COX biosynthetic pathway of arachidonic acid to PGH₂, showing the chemical structure of the substrate and the metabolites.
FIG. 4 is a diagram of the chemical structure for arachidonylethanolamide.
FIG. 6 is a general diagram of enzymatic oxidation of prostaglandin (or thromboxane) ethanolamide (1) and enzymatic hydrolysis of prostaglandin (or thromboxane) ethanolamide (2).

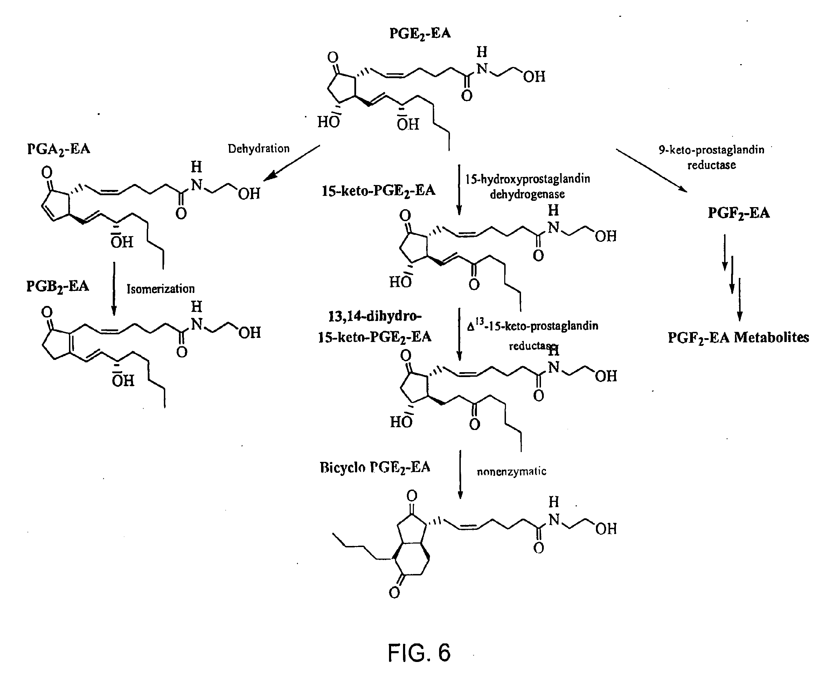

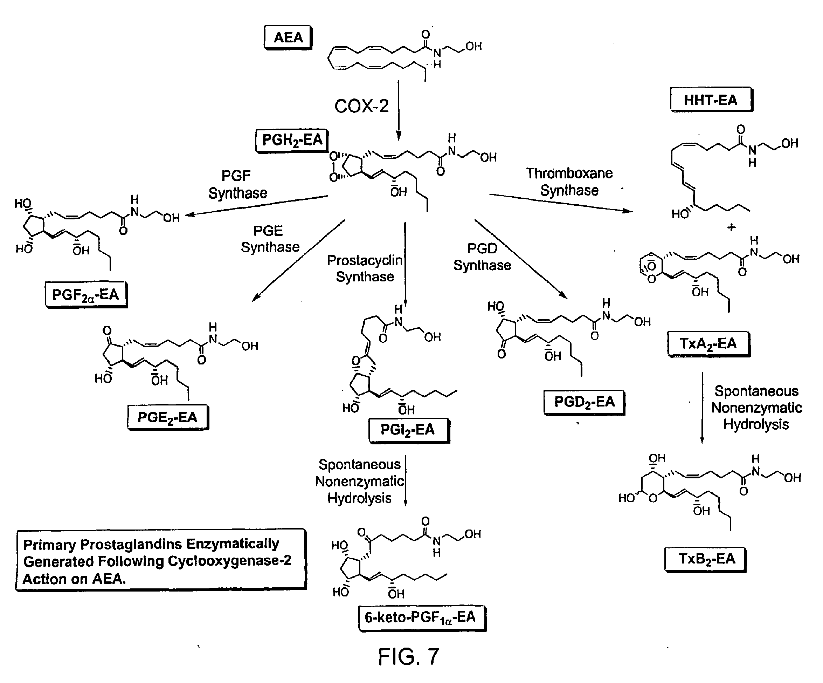

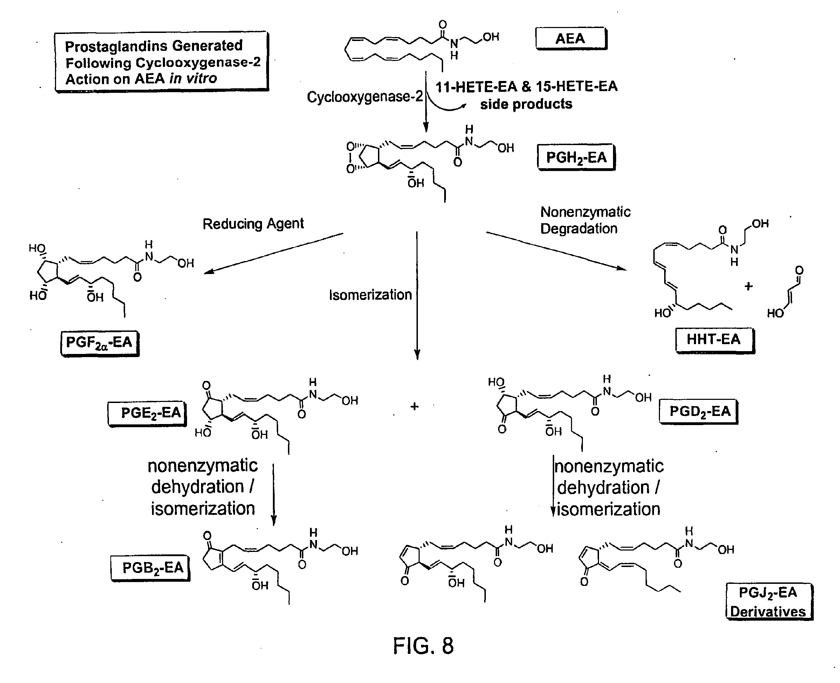

FIG. 6 is a diagram of certain metabolic pathways of PGE₂-EA illustrating a selection of the type-specific enzymatic and nonenzymatic biotransformations that prostaglandin ethanolamides (PG-EAs) and thromboxane ethanolamides (Tx-EAs) undergo. This diagram of PGE₂-EA biotransformations can be similarly constructed for other PG-EAs and Tx-EAs (see, FIGs. 7 and 8). Also, prostaglandin and thromboxane biotransformation pathways can be used as a pattern by one of ordinary skill in the art for the same purpose, in light of the present invention.
FIG. 7 is a diagram of the prostaglandins enzymatically generated following COX-2 action on AEA and in the presence of enzymes downstream of COX-2 (for instance, certain *in vivo* environments).
FIG. 8 is a diagram of certain prostaglandins generated following COX-2 action on AEA *in vitro.*

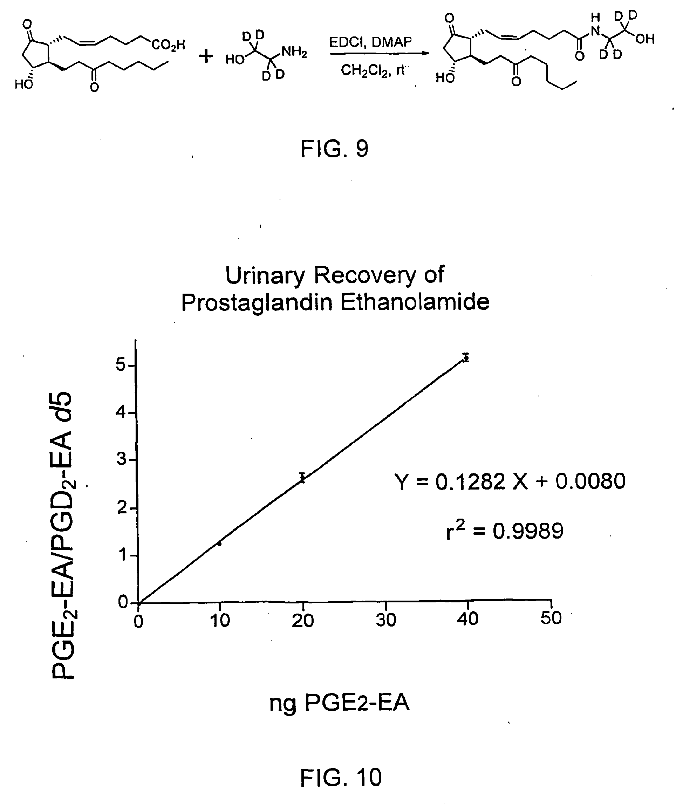

FIG. 9 is a diagram of tetradeuterated prostaglandin or thromboxane ethanolamide standards.
FIG. 10 is a graph showing the recovery of PGE₂-EA from human urine.

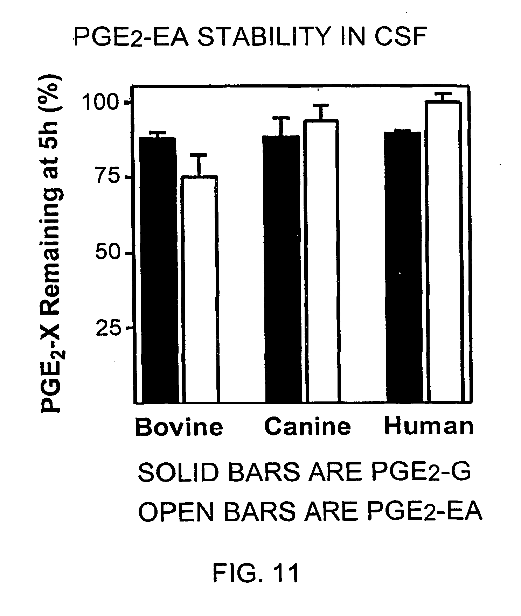

FIG. 11 is a graph showing the stability of PGE₂-EA incubated for 5 hours at 37°C in bovine, canine, and human cerebrospinal fluid.

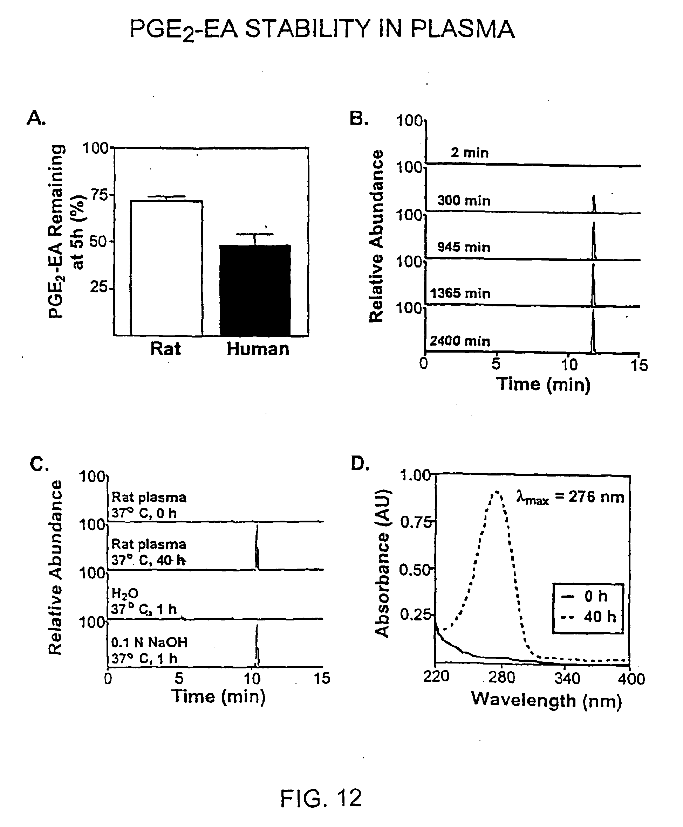

FIG. 12 (A) is a graph showing the stability of PGE₂-EA in plasma. The PGE₂-EA (4 mg/mL) remaining after incubation in rat or human plasma for 5 h at 37° C (mean ± S.E., n³ = 3). (B) PGE₂-EA (4 mg/mL) is incubated in rat plasma for the indicated times and subjected to LC/MS analysis with selected ion monitoring for the mass corresponding to the dehydration product of PGE₂-EA (m/z 400). Chromatograms are normalized to the 2 minute sample. (C) PGE₂-EA (4 mg/mL) are treated as indicated and samples are analyzed for the dehydration product by LC/MS (m/z 400). Chromatograms are normalized to the base treated sample (lowest panel). (D) Is a graph of the absorption spectra of PGE₂-EA (20 mg/mL) incubated in rat plasma for the indicated times.

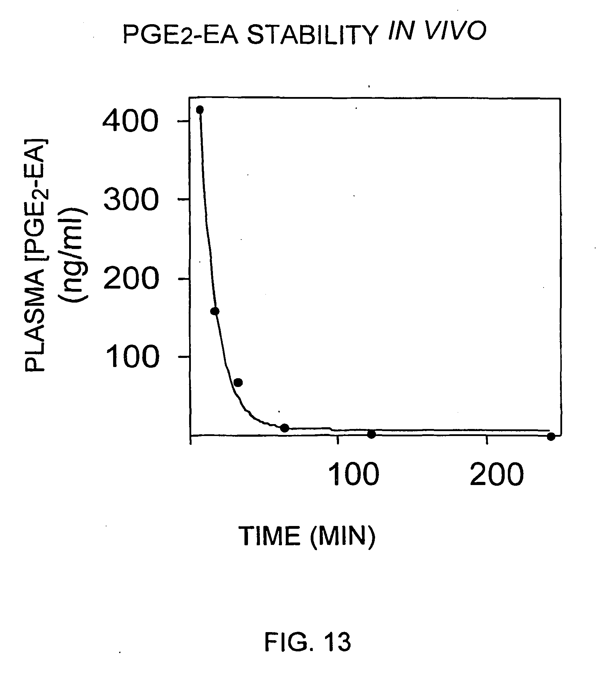

FIG. 13 is a graph showing the in vivo pharmacokinetics of PGE₂-EA in a rat given 2 mg/kg PGE₂-EA by IV. The calculated plasma half life of PGE₂-EA is 380 ± 50 seconds (n = 3). A large apparent volume of distribution was observed (300-400 mL) and both PGE₂-EA and 13,14-dihydro-15-keto-PGE₂-EA were routinely detected at 2 hours post-dosing.

### 4.0 BEST MODE FOR CARRYING OUT THE INVENTION

The present invention provides methods for detecting and measuring cyclooxygenase-2 (COX-2) activity in a sample of a subject thereof. Certain aspects of the present invention focus on detecting and measuring metabolites of AEA, namely, PGE₂-EA, PGD₂-EA and TxA₂-EA. The present invention provides methods for detecting and measuring COX-2 activity, methods for identifying tumors in a subject, evaluating relative tumor severity, and following tumor response to therapy, and methods for detecting inflammation in a subject and evaluating relative inflammation severity.

### 4.1 DEFINITIONS

The term *in vivo* includes the meaning of processes occurring in an animal, in tissue or cell culture, or in samples taken from an animal or culture.

The term *in vitro* includes the meaning of processes occurring in systems wholly or partially purified from the natural environment, such as with purified enzymes or defined enzyme systems.

Purified means partially or wholly isolated away from the natural milieu of factors normally associated with a particular macromolecular species. In certain embodiments, the purified factor comprises 50 percent or more (on a molar basis) of all macromolecular species present in the isolated form. In certain embodiments, a purified composition will comprise more than about 80 percent of all macromolecular species present. In certain preferred embodiments, a purified composition comprises more than about 90 percent of all macromolecular species present. Most preferably, the object species is purified to essential homogeneity (contaminant species cannot be detected in the composition by conventional detection methods) wherein the composition consists essentially of a single macromolecular species. Solvent species, small molecules (<500 Daltons), and elemental ion species are not considered macromolecular species. In non-liquid compositions, "purified" is based upon dry weight and the same percent purities stated above are embodied.

A COX-2 selective substrate is a substrate that is transformed to an enzymatic reaction product by the COX-2 enzyme; but is not transformed, or is not significantly transformed, to a reaction product by the COX-1 enzyme. It is most preferred that a COX-2 selective substrate of the present invention is not enzymatically transformed to a reaction product by COX-1. In certain embodiments, COX-1 may have some activity on the COX-2 selective substrate, but it is not significant relative to the COX-2 activity. Relatively insignificant activity can be determined, for example, by measuring the ratio of substrate oxygenation using purified COX-1 and COX-2.

In certain embodiments, the ratio of COX-1 activity *versus* COX-2 activity for a COX-2 selective substrate, expressed as a percentage, is about 50% or less; in certain embodiments, 40% or less; in certain embodiments, 30% or less; in certain embodiments, 25% or less; in certain embodiments, 20% or less; in certain embodiments, 10% or less; in certain embodiments, 5% or less; in certain embodiments, 3% or less; in certain embodiments, 2% or less; and in certain preferred embodiments 1% or less. The lower the percentage (above), the more preferred the embodiment. A highly preferred COX-2 selective substrate is metabolized by COX-2, but is not metabolized by COX-1.

The terms "COX-2 specific substrate" and "COX-2 selective substrate" are used interchangeably herein.

In general, enzyme activity refers to the rate at which substrate is consumed or product is formed in an enzymatic reaction under a given set of reaction conditions. The Standard International (SI) unit for enzyme activity is an enzyme unit (U) and is defined as the amount of enzyme needed to produce 1 µmole product/minute. A unit may be defined differently herein (*e.g.*, the amount of enzyme needed to produce 1 nmole product per minute or the amount of enzyme needed to consume 1 µmole substrate per minute). Additional determinations of enzyme activity can be compared when utilizing similar or preferably identical reaction conditions. It is understood that reaction conditions can be changed and a new enzyme activity scale determined (*e.g.*, by generating a standard curve of enzyme activity and use thereof, a process which is known to one of ordinary skill in the art). The specific activity of a particular enzyme preparation refers to the total enzyme units divided by the total amount of protein present in the preparation. A preferred unit of specific activity is U per mg of protein (U/mg).

As used herein, references to COX include both COX-1 and COX-2.

Arachidonyl ethanolamide (AEA) is defined herein to be a COX-2 selective substrate.

The terms "arachidonyl ethanolamide" and "anandamide" are used interchangeably herein.

As used herein, "prostaglandin ethanolamides" (PG-EAs) are included in the meaning of COX-2 selective metabolites.

Certain abbreviations include: cyclooxygenase (COX), cyclooxygenase-1 (COX-1), cyclooxygenase-2 (COX-2), prostaglandin (PG), prostaglandins (PGs), ethanolamide (EA), prostaglandin ethanolamides (PG-EAs), arachidonyl ethanolamide (AEA), prostaglandin-ethanolamide (PG-EA), thromboxane (Tx).

As defined herein and using the abbreviations above, PG-EAs can include, but are not limited to: PGB₂-EA, PGD₂-EA, PGE₂-EA, PGF₂α-EA, TxB₂-EA, 6-keto-PGF₁α-EA, 15-keto-PGE₂-EA, 13,14-dihydro-15-keto-PGE₂-EA, PGG₂-EA, PGH₂-EA, PGA₂-EA, PGJ₂-EA, PGJ₂-EA derivatives, bicyclo-PGE₂-EA, TxA₂-EA and PGI₂-EA (also referred to herein as prostacyclin-EA).

Tumor type typically references the tissue of tumor origin, but can also refer to the current tissue in which a tumor is located (*e.g.*, colon cancer, liver cancer, or pancreatic cancer). The stage and grade of a tumor is related to severity and medical definitions of stages and grades within a continuum are known in the art for each tumor or cancer type. Each specialty within oncology (*e.g.*, hematology, colorectal, liver, pancreatic, lung, brain, dermatology, etc.) may have a particular standard for the stage and grade scale of the tumors used within that clinical specialty, known to one of skill in that art, which varies from the general definitions of tumor stage and grade provided below.

Tumor grade is determined by the appearance of the tumor under the microscope and how quickly the tumor is likely to grow and spread. In general, grading systems are different for each type of cancer, but are known to one of ordinary skill in the art. For example, grade I tumors are the least malignant appearing, grade II tumors are moderately differentiated with a moderately malignant appearance, grade III tumors are less differentiated and show enhanced signs of tissue invasion, and grade IV tumors display the least differentiation and are the most malignant appearing. The grade of a tumor is determined by one of ordinary skill in the art.

The stage of a tumor refers to the extent of a cancer, how advanced the tumor is in the patient (*e.g.*, whether the disease has spread from the original site to other parts of the body). The stage of a tumor is generally determined by radiographic studies such as a computed tomography (CT) scan, magnetic resonance (MRI) imaging and/or ultrasound. Tumor staging is determined by one of ordinary skill in the art and can vary by tumor type or as a field advances, standard staging practices may change. Certain definitions of stages for various cancers are provided in the Dictionary of Cancer Terms on CANCERNET which is a service of the National Cancer Institute available on the World Wide Web at "http://cancernet.nci.nih.gov/dictionary/dictionaryfull.html". A physical location for inquiry or obtaining a copy of the Dictionary of Cancer Terms is: NCI Public Inquiries Office; Building 31, Room 10A03; 31 Center Drive, MSC 2580; Bethesda, MD 20892-2580.

Staging refers to performing exams and tests to learn the extent of the cancer within the body, especially whether the disease has spread from the original site to other parts of the body.

### 4.2 CYCLOOXYGENASES AND PROSTAGLANDINS

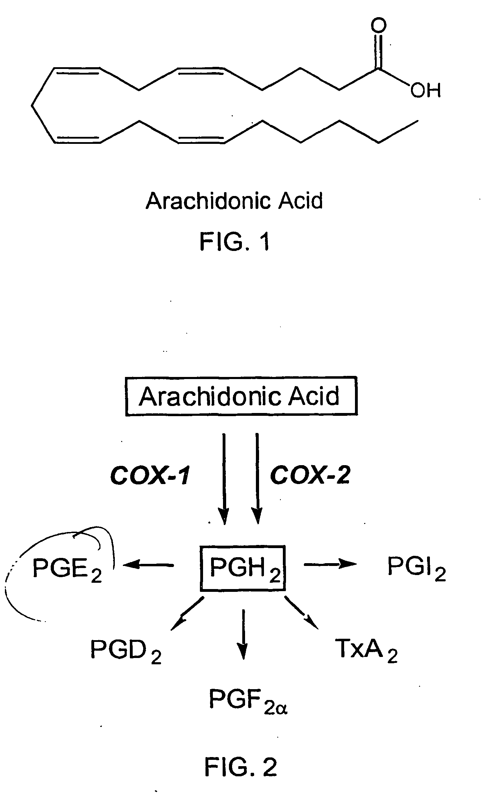

Cyclooxygenase (COX; prostaglandin endoperoxide synthase, EC 1.14.99.1) catalyzes the *bis*-dioxygenation of arachidonic acid (FIG. 1) generating prostaglandin (PG) H₂ (FIGS. 2-3). This is the committed step in prostaglandin and thromboxane biosynthesis. Two isoforms of COX have been cloned from animal cells including constitutively expressed COX-1 (DeWitt, D. L., and Smith, W. L. 1988, Proc. Natl. Acad. Sci. USA, 85:1412-1416; Merlie, et al. 1988, J. Biol. Chem., 263:3550-3553; Yokoyama, et al. 1988, FEBS Lett., 231:347-351; DeWitt, et al. 1990, J. Biol. Chem., 265:5192-5198; and Yokoyama, C. and Tanabe, T. 1989, Biochem. Biophys. Res. Commun., 165:888-894) and inducibly expressed COX-2 (Xie, et al. 1991, Proc. Natl. Acad. Sci. USA, 88:2692-2696; Kujubu, et al. 1991, J. Biol. Chem., 266:12866-12872; O'Banion, et al. 1991, J. Biol. Chem., 266:23261-23267; Hla, T. and Nielson, K. 1992, Proc. Natl. Acad. Sci. USA, 89:7384-7388; Jones, et al. 1993, J. Biol. Chem., 268:9049-9054; and Feng, et al. 1993, Arch. Biochem. Biophys., 307:361-368).

Prostaglandins produced as a result of the activity of COX are known to have numerous physiological functions. These functions include the antithrombogenic action of prostacyclin released by the vascular endothelium and the cytoprotective effect of prostaglandins produced by the gastric mucosa (Whittle, et al. 1980, Nature, 284:271-273). COX-2 is typically expressed following the activation of normal cells and certain atypically proliferating cells, by various pro-inflammatory agents including certain cytokines (Hla, T. and Nielson, K. 1992, Proc. Natl. Acad. Sci. USA, 89:7384-7388; Feng, et al. 1993, Arch. Biochem. Biophys., 307:361-368), endotoxin (Lee, et al. 1992, J. Biol. Chem., 267:25934-25938) and certain mitogens (Kujubu, et al. 1991, J. Biol. Chem., 266:12866-12872; O'Banion, et al. 1991, J. Biol. Chem., 266:23261-23267; and Hla, T. and Nielson, K. 1992, Proc. Natl. Acad. Sci. USA, 89:7384-7388).

Prostaglandins represent a class of substances produced in a wide variety of cells. In general, PGs act on the cells that produce them, on neighboring cells, or over short distances and can be classified as autocrine hormones. PGs and their relatives are usually thought of as potent local hormones (autocrine and paracrine) acting over a short lifetime. PGs, and related compounds, prostacyclin (PGI₂), thromboxanes (TX), leukotrienes (LT), and lipoxins (LP), derive from fatty acids stored in cellular membranes as phospholipids or triglycerides, especially arachidonic acid, with an open chain, 20-carbon structure (FIGS. 1-3). Prostaglandins generally resemble hairpins structurally with a five-membered ring and two chains extending from the ring. In general, substituents on the five-membered ring determine the subclass and activity of the prostaglandins. A series of synthetic reactions catalyzed by enzymes in the membranes, and certain non-enzymatic transformations, culminate in the release of prostaglandin product.

### 4.3 DETECTING COX-2 ACTIVITY BY DETECTING PROSTAGLANDIN ETHANOLAMIDES

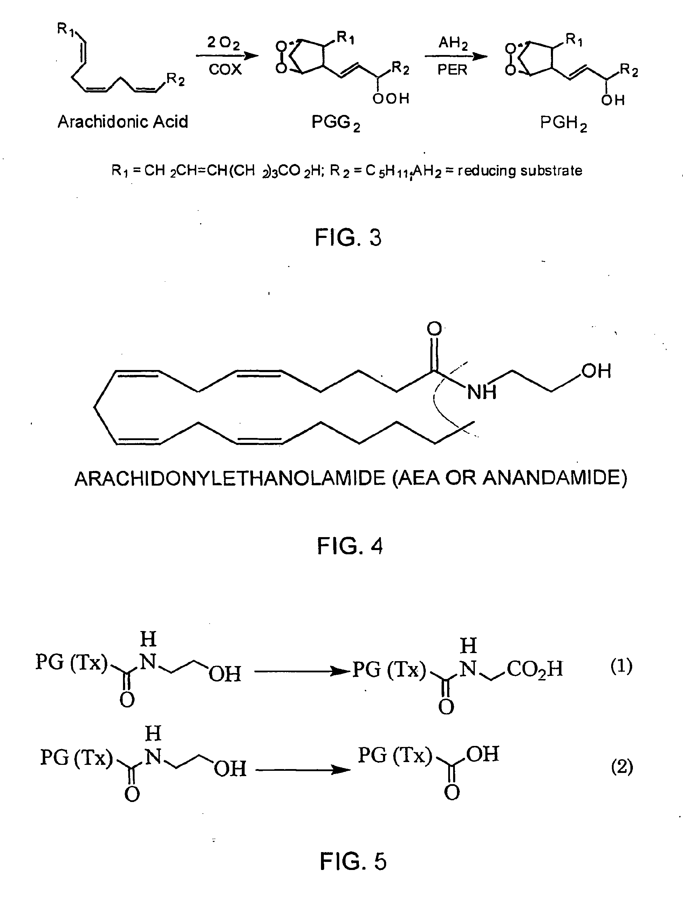

AEA is an unique substrate specific for COX-2 (FIG. 4). COX-2 catalyzes the conversion of AEA to prostaglandin H₂ ethanolamide (PGH₂-EA) (see FIG. 5-8; Yu, M., et al 1997, J. Biol. Chem. 272:21181-21186. The PGH₂-EA is subsequently enzymatically and nonenzymatically metabolized to a variety of compounds, such prostaglandin E₂ ethanolamide (PGE₂-EA) and prostaglandin D₂ ethanolamide (PGD₂-EA). These downstream metabolites will be referred to, herein, as PGH₂-EA metabolites. PGH₂-EA metabolites are PGB₂-EA, PGD₂-EA, PGE₂-EA, PGF₂α-EA, TxB₂-EA, 6-keto-PGF₁α-EA, 15-keto-PGE₂-EA, 13,14-dihydro-15-keto-PGE₂-EA, PGG₂-EA, PGH₂-EA, PGA₂-EA, PGJ₂-EA, PGJ₂-EA derivatives, bicyclo-PGE₂-EA, HETEs, TxA₂-EA and PGI₂-EA., which are metabolized directly from AEA by COX-2, will also be referred to as PGH₂-EA metabolites in this application. In general, all prostaglandin and thromboxane ethanolamides are susceptible to enzymatic oxidation of the ethanolamide moiety and can undergo oxidation (FIG. 5). A representative example for PGE₂-EA is shown in FIG. 6. In addition, individual metabolites can enter the 15-hydroxyprostaglandin dehydrogenase/Δ¹³-15-keto-prostaglandin reductase pathway to generate 13,14-dihydro-15-keto metabolites, for example 15-keto PGA₂-EA and 13,14-dihydro-PGA₂-EA. In general, PGE₂-EA metabolites can undergo dehydration to enter the PGA₂-EA/PGB₂-EA pathway. For example, 13,14-dihydro-15-ketoPGE₂-EA can dehydrate to yield 13,14-dihydro-15-keto-PGA₂-EA and 13,14,-dihydro-15-keto-PGB₂-EA. Finally, oxidative transformation by P450-mediated ω-oxidation (*e.g.*, C-19, C-20) can occur.

It has been shown that AEA is found endogenously (Devane et al. 1992, Science 258:1946-1949, ). The amount of PGH₂-EA metabolites in a biological sample correlates with the COX-2 specific activity in the subject from which the sample was collected. The amount of COX-2 activity is a marker of or a measurement of inflammatory or cancerous disease processes. Certain embodiments of the present invention include methods for making sensitive measurements of picogram quantities of PGH₂-EA metabolites. Further embodiments of the present invention include using these measurements of COX-2 activity to clinically grade or stage a disease process and to assess therapeutic outcomes.

### 4.4 MEASURING PROSTAGLANDIN ETHANOLAMIDES

For the purposes of this application, detecting includes determining if a substance or compound is present in a sample. In the present invention, the substance or compound being detected is a PGH₂-EA metabolite. Detecting can include measuring. In general, measuring means determining the relative or absolute amount of a substance or compound detected. Measurement is generally, but not always, performed relative to a standard. For example, the amount of the standard may be correlated with an amount of COX-2 activity or expression. Therefore, comparing the amount of PGH₂-EA metabolites measured in a sample from a subject indicates an amount of COX-2 activity or expression in the subject.

PGH₂-EA metabolites may be detected and measured in a variety of ways (see Examples 1-6). In certain embodiments of the present invention, for example, a sample is collected from a subject, and a selective COX-2 substrate, such as AEA, is added. Then, a metabolite of the COX-2 specific PGH₂-EA product is measured. In preferred embodiments, downstream metabolites are measured because the half-life of PGH₂-EA in aqueous solution is approximately 20 seconds. Certain downstream metabolites are more stable and are preferred for measurement. A highly preferred downstream metabolite for measurement is PGE₂-EA. In another embodiment of the present invention, for example, both a COX-1 substrate (such as arachidonic acid) and a COX-2 selective substrate (such as AEA) are added to the sample. Then the amount of downstream metabolites derived from the enzymatic actions of COX-1 and COX-2 on each substrate are measured and compared. In both examples, the samples can be incubated with the substrates over time and a series of measurements of metabolites taken and compared in relation to the amount of time that passed. In certain embodiments of the present invention, no substrate is added to the sample. Instead, the PGH₂-EA metabolites derived from the endogenous AEA is detected and measured. A highly preferred downstream metabolite for measurement is 13,14-dihydro-15-keto-PGE₂-EA, a novel lipid formed by the sequential oxidation and reduction of PGE₂-EA by 15-hydroxyprostaglandin dehydrogenase and Δ13,15-keto-prostaglandin reductase. This metabolite is formed *in vivo* following intravenous dosing of PGE₂-EA to rats and is detectable in plasma by the methods described herein. In some cases, AEA is administered to the subject prior to sample collection, followed by sample collection, and detection and measurement of PGH₂-EA metabolites present in the sample. In addition, in preferred embodiments, the amount of PGH₂-EA metabolites measured in the sample is related to an amount of COX-2 activity in the sample or the subject.

Samples from the subject can be processed is several ways (see Examples 5-6). For example, the sample may be extracted at least one time with a solvent, to remove the PGH₂-EA metabolites from the sample for analysis. Extraction can be followed by evaporation. The resulting residue may be redissolved in another solvent and analyzed. This process might involve several rounds of the extraction, evaporation and redissolving steps. Alternatively, the solution resulting from one or more extractions of the sample may be filtered and analyzed. In certain preferred embodiments, the sample is extracted, filtered and analyzed for PGH₂-EA metabolite content.

One aspect of the present invention is a method of detecting COX-2 activity in a biological sample, comprising: incubation of the biological sample with AEA, extracting the sample with a solvent, evaporating the solvent to leave a residue and analyzing the residue for PGH₂-EA metabolites wherein the presence of PGH₂-EA metabolites is indicative of COX-2 activity (for instance, see Examples 1, 2, and 3). In certain embodiments, the amount of PGH₂-EA metabolites is measured and related to the quantity of COX-2, COX-2 expression, or COX-2 activity. In general, the analysis of PGH₂-EA metabolites includes, but is not limited to, detection by liquid chromatography- mass spectrometry (Examples 1-2, 5-6).

Another embodiment of the present invention is a method of detecting COX-2 activity in a biological sample, comprising: extracting PGH₂-EA metabolites present in the sample with a solvent, evaporating the solvent to leave a residue and quantifying PGH₂-EA metabolites in the residue, wherein the presence of PGH₂-EA metabolites is indicative of COX-2 activity. Preferably, the quantity of PGH₂-EA metabolites detected are related to a quantity of COX-2 activity.

Yet another embodiment of the present invention is a method of detecting a COX-2 activity in a biological sample, comprising: extracting the sample at least one time with a first solvent, evaporating the solvent to leave a residue, dissolving the residue in a second solvent, separating at least one PGH₂-EA metabolite from the dissolved residue with a separation device, lyophilizing the separated PGH₂-EA metabolite, dissolving the lyophilized PGH₂-EA metabolite in a third solvent and detecting the dissolved PGH₂-EA metabolite with a detection device, wherein the presence of the PGH₂-EA metabolite is indicative of the COX-2 activity (for instance, see Examples 1, 2, and 3). In certain embodiments, the amount of PGH₂-EA metabolite is measured and related to a quantity of COX-2 protein, COX-2 expression, or COX-2 activity. In general, the separation device for separating the PGH₂-EA metabolite from a residue in the second solvent includes, but is not limited to, a liquid chromatography device.

Yet another aspect of the present invention is a method of detecting COX-2 activity in a biological sample, comprising:
extracting at least one PGH₂-EA metabolite from the sample with a solvent, evaporating the solvent to leave a residue, dissolving the residue in a second solvent, filtering the dissolved residue and
detecting a PGH₂-EA metabolite in the filtered solution with a detection device, wherein the presence of PGH₂-EA metabolite in the sample is indicative of COX-2 activity. Preferably, the detected PGH₂-EA metabolite is measured and the amount of PGH₂-EA metabolite measured is related to a quantity of COX-2 activity.

### 4.4.1 USE OF A STANDARD

In certain embodiments, isotopically labeled PGH₂-EA metabolites, such as PGE₂-EA and TxA₂-EA, are used as an internal standard in quantifying PG-EAs. In certain preferred embodiments, mass spectrometric quantification of PGH₂-EA metabolites is performed. One example of the synthesis of isotopically labeled ethanolamides of prostaglandins and thromboxanes is shown in FIG. 9. In general, isotopically labeled PG- or Tx-EA are prepared by coupling the target PG or Tx free acid with anhydrous, isotopically labeled ethanolamide and purified by chromatography. In another example, labeled standard ethanolamides of prostaglandins and thromboxanes can be labeled by substituting ²H or ³H for a ¹H attached to at least one of the carbons 2-20 of the arachidonyl carbon chain. Alternatively, a PGH₂-EA metabolite can be labeled by substituting ¹³C or ¹⁴C for at least one ¹²C in the arachidonyl carbon chain. Labeled PGH₂-EA metabolites can also be made by reacting labeled AEA with COX-2, followed by purification of the reaction products. In general, labeled PGH₂-EA metabolites may also be synthesized by substituting at least one atom in the molecule with an isotope. In certain embodiments, the isotope is non-positron emitting. In certain embodiments, the isotope is positron emitting. Alternatively, PGH₂-EA metabolites can be derivatized to produce chemiluminescent or fluorescent standards, which can be used, for example, in PGH₂-EA quantitation using high performance liquid chromatographic separation coupled with fluorescence or chemiluminescence detectors.

Labeled PG-EA or Tx-EA standard curves, which correlate with the presence or absence of cancer in a patient, can be generated. For example, this can be accomplished by collecting samples from groups of patients with different types, stages, or grades of cancer, measuring the amount of a PGH₂-EA metabolites in the samples, and graphing the amounts of the PGH₂-EA metabolite *versus* the types of cancer. In certain embodiments, types can be varieties. In preferred embodiments, the types are a range of particular cancers measured on a clinical scale of severity or aggressiveness. Such a standard curve may indicate that the larger the amount of cancer or the more progressed the cancer in the patient, the more PGH₂-EA metabolite found in the sample from the patient. In addition, since small amounts of PGH₂-EA metabolites can be found in samples collected from people without cancer, a threshold level of PGH₂-EA metabolites will be observed at which amounts of PGH₂-EA metabolites below the threshold will correlate with the lack of cancer in the patient. On the other hand, for example, amounts of PGH₂-EA metabolites measured in a sample that are above the threshold level correlate with the presence of cancer in the patient.

In a similar example, a standard curve can be developed which correlates the amount of PGH₂-EA metabolites observed in a sample from a patient with an amount of inflammation. Such a standard curve can be generated by collecting samples from a patient population suffering from inflammatory processes, and correlating the amounts of PGH₂-EA metabolite in the samples with the severity of disease in the patient population.

In another example, no standard or standard curve is used. Instead, a series of samples may be collected from a single patient over a period of time, and the amount of PGH₂-EA metabolite in each sample measured. Then the amounts of PGH₂-EA metabolite measured would be compared and correlated with the amount of time which had passed between sample collections. If the amount of PGH₂-EA metabolite in the samples increased over time, for example, this would indicate that the patient's disease state is worsening, or that therapeutic intervention is not effective. On the other hand, if the amount of PGH₂-EA metabolite measured in the samples decreased over time, for example, this would indicate that the patient's disease state is improving or that therapeutic intervention is successful.

### 4.4.2 DETECTION AND MEASURING DEVICE

In general, a detection device for detecting PGH₂-EA metabolites includes, but is not limited to, a mass spectrometer, a chromatography-coupled mass spectrometer, an immunoassay or an enzyme-linked immunoassay, or other means for detecting PGH₂-EA metabolites known in the art (see Examples 1-6). For example, in certain embodiments of the present invention, liquid chromatography/mass spectrometry (LC/MS) is conducted, preferably with a Waters 2690 Separations Module with a Zorbax RX-C18 narrow bore column (15 cm x 2.1 mm, 5 µm) interfaced to a Finnigan TSQ-7000 triple quadrupole mass spectrometer. Sodiated analytes are eluted with increasing concentrations of MeCN in 0.001% aqueous sodium acetate. Evaluation of PGH₂-EA metabolites in biological samples is conducted with selected ion monitoring and quantification is accomplished using tetradeutereated standards, such as tetradeutereated PGE₂-EA. Electrospray ionization (ESI) is carried out using nitrogen as sheath (76 psi) and auxiliary gas (14 psi) to assist with nebulization. A potential of 5.5 kV is applied to the ESI needle and the capillary temperature is maintained at 220 °C. Mass spectral parameters are optimized to obtain maximum sensitivity without sacrificing unit resolution.

### 4.4.3 SEPARATION DEVICE

A variety of separation devices known in the art for separating prostaglandins from a sample may be used. In general, separation devices include, but are not limited to, extraction columns, affinity columns, filters, thin-layer chromatography plates and gels.

In certain embodiments of the present invention, a PGH₂-EA metabolite in a sample may be isolated or purified (separated partially or substantially from the natural constituents of a PGH₂-EA metabolite containing sample) using one or more techniques known in the art for the separation of chemical and especially prostaglandin compounds, for example, but not limited, to liquid chromatography.

### 4.4.4 SUBJECTS

In certain embodiments of the present invention, the subject includes a mammal, such as a rodent, preferably a human, or a cultured cell of a mammal, including a cultured cell of a human. Other subjects include farm animals and show animals (horses, cattle, sheep, pigs and swine, goats, fowl, and the like), pets (dogs, cats, parrots, canaries and the like), animals kept in zoos and endangered species (elephants, lions, tigers, antelope, zebra, anteaters, water buffalo, pandas, cheetahs, kangaroos, ostriches, eagles, condors, finches, and the like) or a cultured cell of said animal.

### 4.4.5 SAMPLES

In certain embodiments, the sample is urine or may be collected from or processed from urine. PGH₂-EA metabolites can be measured in urine by isotope dilution mass spectrometry. A fixed volume of urine is treated with an appropriate internal standard (*e.g.*, tetradeutereated PGE₂-EA) and then loaded on reversed-phase extraction cartridges. The sample is then washed (*e.g.*, 1 ml pH 4.0 20 mM sodium acetate) and PGH₂-EA metabolites, including the added internal standard, are eluted with organic solvent (*e.g.*, two 1 ml aliquots of MeCN). The solvent is evaporated and the residue is analyzed by LC/MS. A typical procedure wherein synthetically generated PGE₂-EA and *d5*-PGD₂-EA is added to fresh human urine is depicted in FIG. 10.

PGH₂-EA metabolites are stable in cell culture, bovine, canine and human CSF (FIG. 11) and human and rat plasma (FIG. 12) incubated 5 hours at 37°C. FIG. 13 shows the in vivo pharmacokinetics of PGE₂-EA in a rat given 2 mg/kg PGE₂-EA intravenously. The calculated half-life of PGE₂-EA is 380 ± 50 seconds (n=3). Notably, this half-life greatly exceeds that observed for the free acid PGE₂ *in vivo*, highlighting one significant advantage of the methods described herein over previously described methods for COX activity assessment *in vivo*. A large apparent volume of distribution was observed (300-400 ml) and both PGE₂-EA and 13,14-dihydro-15-keto- PGE₂-EA were routinely detected at 2 hours post-dosing. In certain embodiments, the sample is blood, plasma, cerebrospinal fluid, saliva, sputum, bile, joint fluid, biopsy, immune cells, cancer cells, tumor cells, malignant cells, inflammatory cells, non-tumor cells, non-immune cells, cells not activated by inflammatory stimuli or may be collected from or processed from one or more of these fluids and tissues. In certain embodiments of the present invention, the sample may be conditioned media from a cell culture. In certain embodiments of the present invention, a plurality of samples may be collected from the subject, with a period of time being allowed to pass between consecutive collections of the samples. The amounts of PGH₂-EA metabolites present in these samples are measured, compared and related to the periods of time that had been allowed to pass between collections. In certain embodiments, the subject is a patient and the samples are taken in order to evaluate the effectiveness of anti-cancer therapy and to evaluate tumor state, severity or load in the patient.

In general, samples can be collected from or prepared from cultured cells (Example6). A variety of cells lines known to one skilled in the art are acceptable. In addition, primary cell cultures can be used. Methods for collecting and culturing primary cell cultures are well known in the art.

### 4.4.6 ANTIBODY SYNTHESIS

Monoclonal and polyclonal antibodies to PGH2-EA metabolites or their metabolites can be made using standard antibody generation techniques in light of the present invention (Cohen, et al., U.S. Patent Serial No. 5,589,575 ; McCafferty, et al. 1996, Antibody Engineering, a Practical Approach, IRL Press; Mernaugh & Mernaugh 1994, *Methods for the Production of Monoclonal Antibodies,* in **Molecular Methods in Plant Pathology**). For example, monoclonal antibodies against PGE₂ are commercially available from Cayman Chemical (118 E. Ellsworth Rd., Ann Arbor, MI 48108, 800-364-9897) and chemiluminescent ELISA kits for several PGs, HETE and TxB₂ are available from Assay Designs, Inc. (800 Technology Dr., Ann Arbor, MI 48108; 734-668-6113). To create antibodies that recognize PGE₂-EA, for example, the cyclopentyl substituents and amide moieties of the PGE₂-EA are protected using standard techniques. Then the protected PGE₂-EA is chemically linked to an appropriate conjugate (e.g., keyhole limpet hemocyanin (KLH)) preferably via covalent attachment at carbon 15. The haptenized product is deprotected and the resultant immunogen is used to inoculate mice, rabbits, or goats using standard techniques. According to standard protocols, serum would be collected from the immunized animals and antibodies are purified from the serum. Alternative antibody development strategies using the same immunogen could also be employed (single-chain antibodies, hybridomas, etc.).

### 4.5 COX-2 AND INFLAMMATORY DISEASES/DISORDERS

A wide variety of human diseases are associated with inflammation. These range from acute appendicitis to asthma, myocardial infarction, specific immunological disease processes, infection with viruses or bacteria, malignancy and metastasis, endotoxemia and reperfusion injury. Since COX-2 activity is important in the progression of these diseases, the present invention is a useful method of diagnosing or monitoring disease state. The present invention is also useful in detecting and treating non-malignant or immunological-related cell-proliferative diseases such as psoriasis, pemphigus vulgaris, Behcet's syndrome, acute respiratory distress syndrome (ARDS), ischemic heart disease, post-dialysis syndrome, leukemia, acquired immune deficiency syndrome, septic shock and other types of acute inflammation, and lipid histiocytosis. Essentially, the present invention can be used to facilitate the detection, measurement and treatment of any disorder which is etiologically linked to the inflammatory process (for instance, see Example 4).

For example, in certain aspects of the present disclosure, a sample is collected from a patient suspected of having an inflammatory disease or diagnosed with an inflammatory disease and PGE₂-EA in the sample is detected and measured. The amount of PGE₂-EA present in the sample correlates with COX-2 activity in the patient and is a marker of the progress or severity of that disease or disease process. In another example, a series of samples are collected from the patient over a period of time. During that period of time, the patient undergoes treatment for the inflammatory disease. Changes in the amount of PGE₂-EA measured over time are indicative of changes in COX-2 activity and changes in the patients disease state. This information would be used by the physician to evaluate the patient's condition as well as the effectiveness of therapeutic intervention, wherein a decrease in the PGH₂-EA metabolites or COX-2 activity is indicative of an improvement in the patient's condition or effective therapy.

### 4.6 COX-2 AND CANCER

Studies in human colon cancer have shown that COX-2 expression is increased in colon cancer cells compared to the adjacent colonic mucosa; similar observations have been made in experimental models of colon cancer (Eberhart, CE, et al. 1994, Gastroenterology 107:1183; Sheng, H, et al. 1997, J. Clin. Invest 99:2254; DuBois, RN, et al. 1996, Gastroenterology 110:1259). COX-2 expression is a marker for the metastatic potential of colon cancer cells and is related to patient survival (Tsujii, M, et al. 1997, Proc. Natl. Acad. Sci. USA 94:3336; Sheehan, KM, et al. 1999, JAMA 282:1254). In one study, for example, COX-2 expression was determined in 76 patients with a variety of stages of colorectal cancer (Sheehan, KM, et al., 1999, JAMA 282:1254). Such studies can be used to generate a standard curve for COX-2 expression in cancer and colon cancer in particular (see *supra*)*.* Ten-year survival was significantly higher in patients with the lowest levels of COX-2 expression (68 versus 35 percent). These findings suggest that COX-2 activation promotes tumor growth. Consistent with this hypothesis is a study in which human colon cancer cells that expressed high levels of COX-2 were implanted into nude mice. Treatment with a selective COX-2 inhibitor reduced tumor formation by 85 to 90 percent and inhibited colony formation of cultured cells (Sheng, H, et al. 1997, J. Clin. Invest 99:2254). This benefit was not seen with tumor cells that lacked COX-2.

Certain aspects of the present invention include methods of detecting a tumor in a patient in need thereof, comprising: detecting at least one PGH₂-EA metabolite in a sample obtained from a patient (for instance, see Example 5). The presence of the PGH₂-EA metabolite in the sample is a marker for the presence of the tumor in the patient. More preferably, an amount of PGH₂-EA metabolite detected in the sample of the patient will be measured, wherein the amount of PGH₂-EA metabolite measured is indicative of the amount or severity of tumor present in the patient.

A further aspect of the present invention is a method of measuring and monitoring the size, grade, and/or stage of a tumor, comprising: measuring the amount of PGH₂-EA metabolites in a first sample collected from the subject. Then a period of time is allowed to pass, during which the subject may, or may not, undergo anti-cancer therapy. After the period of time has passed, the amount of PGH₂-EA metabolites in the second sample collected from the subject is measured. The amounts of the PGH₂-EA metabolites in the first and second samples are compared, wherein the difference between the amounts of PGH₂-EA metabolites in the two samples is indicative of changes in the metabolism of the cancer cell.

In certain embodiments of the present invention, a plurality of samples may be collected from the subject, with a period of time being allowed to pass between consecutive collections of the samples. The amounts of PGH₂-EA metabolites present in these samples are measured, compared and related to the periods of time that had been allowed to pass between collections. In certain embodiments, the subject is a patient and the samples are collected in order to evaluate the effectiveness of anti-cancer therapy and to evaluate tumor severity or tumor load in the patient.

In general, the effectiveness of the anti-cancer therapy is evaluated based on the changes in the amount of sample PGH₂-EA metabolite observed over time. For example, increases in the amount of PGH₂-EA metabolites over time indicate continued tumor growth and failure of therapeutic intervention; whereas decreases in the amount of PGH₂-EA metabolites over time are indicative of therapeutic success and tumor regression. In certain embodiments of the present invention, the sample is a culture of cancer cells used as an experimental model or a culture of cancer cells taken from a patient. The cultured cancer cells may be treated with an anti-cancer therapy *in vitro* in order to evaluate the effectiveness of that therapy in relation to alternative cancer therapies. In certain embodiments, this procedure is done in order to determine an optimal anti-cancer therapy for that individual patient.

In certain embodiments, the attending health professional may characterize both an inflammatory process and a malignancy in the subject by detecting or measuring an amount of a prostaglandin ethanolamide in a sample of a subject specifically produced by the offending malignancy and inflammatory lesion.

### 4.7 COX-2 AND RESEARCH

In the prior art, investigations which attempt to identify links between COX-2 expression/activity and disease processes are time- and labor-intensive and often require examination of tissue samples post-mortem. For example, attempts to study the role of COX-2 in Alzheimer's disease (AD) require post-mortem collection of brain tissue from both deceased AD and control subjects and quantitative assessment of COX-2 expression in this tissue using standard biochemical techniques (*e.g*., Western blotting). Such studies are also hampered by the inability to assess enzyme activity, which may or may not correlate with enzyme expression. The use of PGH₂-EA metabolite quantification in this setting allows for a relatively non-invasive quantification of COX-2 activity in vivo. This technique provides at least two fundamental benefits. First, the noninvasive nature allows for much broader testing increasing the sample size in these studies, and permitting rapid and statistically significant association (or lack thereof) between COX-2 activity and the pathology under study. Second, given the possibility of testing patients before disease signs are evident will allow for assessing the role of COX-2 in disease development and progression, in contrast to post-mortem studies which evaluate the role of COX-2 long after the disease process began. Quantification of PGH₂-EA metabolites *in vivo* provides a simple assay for assessing the *in vivo* efficacy of newly developed COX-2 inhibitors.

In addition, there is a great need to discover and develop new COX-2 specific regulators. However, current methods are time and labor intensive. The present invention provides compositions, methods and kits for screening candidate molecules for their ability to regulate COX-2 using cultured cells, tissues, or whole animals (Examples 9-10).

### 4.8 KITS

Certain embodiments of the present disclosure provide an article of manufacture for the detection and/or measurement of COX-2 activity by the detection and/or measurement of PGH₂-EA metabolites by radioassay or immunoassay, comprising an antibody and a set of instructions delineating a process for relating a detection and/or measurement of PGH₂-EA metabolites in a sample to a detection and/or measurement of COX-2 in a subject or a samples thereof. Preferably, the article of manufacture further comprises an antibody against PGH₂-EA metabolites. More preferably, the article of manufacture further comprises the standard reagents required to perform an immunoassay, such as buffers, multi-well plates, additional antibodies and the like. Still more preferably, the article of manufacture further comprises one or more solid phase extraction columns for the isolation/purification of PGH2-EA metabolites. Preferably, the article of manufacture further comprises a set of standards. More preferably, the article of manufacture further comprises an unlabeled PGH₂-EA metabolite internal standard for standard curve development.

In further embodiments the present disclosure provides an article of manufacture or the detection and/or measurement of COX-2 activity by mass spectrometry, which comprises: a set of instructions delineating a process for relating a detection and/or measurement of PGH₂-EA metabolites in a sample to a detection and/or measurement of COX-2 in a subject or a sample thereof and a C18 solid phase extraction column. Preferably, the article of manufacture further comprises a set of standards. More preferably, the article of manufacture further comprises an unlabeled PGH₂-EA metabolite positive control, and a tetradeuterated PGH₂-ERA metabolite internal standard.

### 5.0 EXAMPLES

### 5.1 Example 1

In general, COX-2 enzymatic products can be detected or measured by a variety of methods. For example, reversed phase liquid chromatography-mass spectrometry is used to detect or measure COX-2 enzymatic products. Selected-ion mass chromatograms of sodium adducts of oxygenated AEA metabolites can be generated by monitoring *m*/*z* = 386 (HETE-EAs), 418 (PG-EAs), and 436 (Tx-EA and 6-keto-PGF1α-EA). For example, products are eluted with a 15-minute gradient of 20% to 100% acetonitrile and H₂O (0.001% sodium acetate). LC/MS reveals the presence of five primary products in AEA/COX-2 reaction mixtures. Two closely eluting polar products each display *m*/*z* 418 consistent with the non-enzymatic PGH₂-EA isomerization products PGE₂-EA and PGD₂-EA. Tetradeutereated standards of PGE₂-EA and PGD₂-EA are synthesized and coeluted with these two polar products under multiple chromatographic conditions. An intermediate polarity product with *m*/*z* 346 is observed that is consistent with the PGH₂-EA degradation product HHT-EA. Finally, two closely eluting non-polar AEA metabolites are detected with an *m*/*z* of 386, consistent with the ethanolamides of HETEs (hydroxy-eicosatetraenoic acids that are minor products of arachidonic acid oxygenation by COX-2).

### 5.2 Example 2

Cellular COX-2 enzymatic activity can be determined by exposing cells to exogenous AEA and measuring the COX-2 specific products. For example, unactivated RAW264.7 cells (a murine macrophage cell line) express no detectable COX-2 and low levels of COX-1, but IFN-γ and LPS induce COX-2 expression (Wadleigh, D. J. et al, 2000, J. Biol. Chem. 275:6259-6266). The major arachidonic acid metabolite in these cells is PGD₂, so RAW264.7 cells permit the simultaneous evaluation of AEA oxygenation to PGH₂-EA as well as endoperoxide metabolism by PGD synthase (Landino, L. M., Crews, B. C., Timmons, M. D. et al., 1996, Proc. Natl. Acad. Sci. USA 93:15069-15074). PGH₂-EA metabolites are not detectable in the medium from unactivated RAW264.7 cells following addition of AEA (20 µM) (Kozak, *et al.* 2000, *supra*)*.* However, treating cells with LPS (1 µg/mL) and IFN-γ (10 units/mL) results in the synthesis and release of copious amounts of PGD₂-EA, following the addition of AEA (20µM). A LC-mass spectrogram showing the PGD₂-EA production and extracellular release by activated Raw264.7 macrophages can be generated. Products are eluted with a 15-minute gradient of 20% to 100% acetonitrile in H₂O (0.001% sodium acetate). Chromatograms are normalized to total ion current of AEA- treated activated macrophages. PGD₂-EA biosynthesis is inhibited both by indomethacin (3 µM) and the highly selective COX-2 inhibitor, 2-[1-(4-chlorobenzoyl)-5-methoxy-2-methyl-1*H-*indol-3-yl]-*N*-phenethylacetamide (indomethacin phenethylamide, 3 µM). No significant endogenous PGD₂-EA biosynthesis is detected in the absence of exogenous substrate under these conditions.

### 5.3 Example 3

A sample may be urine or may be collected from or processed from urine. PGH₂-EA metabolites can be measured in urine by isotope dilution mass spectrometry. A fixed, volume of urine is treated with an appropriate internal standard (*e.g.,* tetradeutereated PGE₂-EA) and then loaded on reversed-phase extraction cartridges. The sample is then washed (*e.g*., 1 ml pH 4.0 20 mM sodium acetate) and PGH₂-EA metabolites including the added internal standard are eluted with organic solvent (*e.g*., two 1 ml aliquots of MeCN). The solvent is evaporated and the residue is analyzed by LC/MS. A typical procedure wherein synthetically generated PGE₂-EA and *d4*-PGD₂-EA is added to fresh human urine is depicted in FIG. 10 and demonstrates that this technique provides precise and linear quantitation over the tested range (0-40 ng PGE₂-EA per ml urine).

### 5.4 Example 4

COX-2 expression and activity are generally linked with the inflammatory process, which accompanies a plethora of pathologies including, but not limited to, arthritis/arthropathy, infectious disease, neurodegenerative disease, neoplasia and autoimmune disease. The quantification of prostaglandin PGH₂-EA metabolites from biological fluids obtained non-invasively (*e.g*., blood, urine) will allow for the assessment of COX-2 activity in vivo, reflecting both inflammation and disease severity. In addition, serial testing will allow for the tracking of the natural course of the disease as well as the efficacy of anti-inflammatory therapy. A model for this application would be the ubiquitous use of C-reactive protein (CRP) in the diagnosis and assessment of diseases associated with inflammation. The benefits of using PGH₂-EA metabolites in this context instead of more traditional diagnostic markers, such as CRP, involve the highly specific nature of PGH₂-EA metabolite production. PGH₂-EA metabolites are elevated only when COX-2 activity is elevated whereas CRP elevations, for example, are very non-specific.

Scenario: Elderly woman seeks medical attention for recent onset of joint pain in hands. Urine is collected and PGH₂-EA metabolite quantification is conducted. PGH₂-EA metabolites are elevated supporting a diagnosis of rheumatoid arthritis. COX-2 inhibitor therapy is initiated (*e.g*., celecoxib). After one week, symptoms are only mildly relieved and another urine sample reveals PGH₂-EA metabolite levels are still elevated, indicating that the inflammatory process is still active. Following dosage increase, symptoms are relieved and urinary PGH₂-EA metabolites are normalized.

### 5.5 Example 5

COX-2 expression and activity are linked to several solid tumors, most notably colorectal adenocarcinoma. The quantification of PGH₂-EA metabolites from biological fluids, described herein, provides a noninvasive "early-warning" for clinically undetectable neoplasia. In addition, serial testing following diagnosis will allow for the tracking of the natural course of the cancer as well as the efficacy of antineoplastic therapy. A model for this application would be the use of prostate specific antigen (PSA) in the diagnosis and assessment of prostate adenocarcinomas. The benefits of PGH₂-EA metabolite quantification in this context include (a) relative noninvasiveness, (b) sensitivity (most cancers are advanced once symptomatic) and (c) cost (simple lab diagnostic technique versus colonoscopy for example).

Scenario: Elderly asymptomatic man receives annual physical examination. Urine and blood are collected and PGH₂-EA metabolite quantification is conducted. PGH₂-EA metabolites are elevated in both the urine and plasma, prompting a more detailed search for possible neoplasia. Colonoscopy reveals a single tumor in the descending colon which, following biopsy, proves malignant. Standard chemotherapy is initiated. Following treatment, urinary and plasma PGH₂-EA metabolite levels have normalized. Annual colonoscopies for 3 years reveal no recurrence. After 3 1/2 years, patient visits physician for an unrelated reason and urinary PGH₂-EA metabolites are quantified. PGH₂-EA metabolite levels are markedly elevated, indicating the recurrence of carcinoma. The physician recommends colonoscopy, which reveals the presence of carcinoma. Aggressive chemotherapy is initiated and urinary PGH₂-EA metabolite levels are monitored.

### 5.6 Example 6

The measurement of PGH₂-EA metabolites from *in vitro* samples (*e.g*., cell culture, biopsy samples) allows for the direct quantification of COX-2 activity. Current methods which quantity cyclooxygenase activity do not directly distinguish between COX-1 and COX-2. Methods which quantify COX-2 expression (*e.g*., Western blotting) do not assess activity which may or may not correlate with expression levels.

Scenario: Researchers investigating new NSAIDS expose cultured cells expressing COX-2 to various concentrations of test compounds for predetermined periods of time. At the conclusion of the exposures, conditioned medium is collected from each culture. The samples of conditioned medium are assayed for the presence of PGH₂-EA metabolite. The researchers find that most of the test compounds have no significant affect on the production of PGH₂-EA metabolite by the cultured cells. However, one compound ("compound X") dramatically reduces the amount PGH₂-EA metabolite produced by the cultured cells. Therefore, "compound X" inhibits COX-2. The researchers focus their efforts on "compound X," which may become a new COX-2 specific treatment of inflammatory diseases or cancer. In further experiments, the researchers determine that compound X does not inhibit COX-1. Compound X is, therefore, identified as a COX-2 specific inhibitor.

### 5.7 Example 7

In certain embodiments, the present invention can be employed to identify a previously- undescribed small molecule modulator of COX-2 activity. This can be done with the following method. Briefly, RAW264.7 cells at 30-40% confluence are activated with lipopolysaccharide (LPS, 20 ng/mL) and treated with a series of concentrations of a test compound. Cells are incubated for 12 h at 37°C and then medium is removed and replaced with buffered saline. Cells are then treated with 50 µM AEA and incubated an additional 30 min at 37°C. Following incubation, buffered saline is collected and treated with tetradeutereated PGH₂-EA metabolite standard. Buffered saline is extracted twice with equal volumes of 2:1 CHCl₃:MeOH. The combined organic extract is evaporated under a stream of argon. The resultant residue is redissolved in 1:1 H₂O:MeCN and analyzed by liquid chromatography-mass spectrometry (LC-MS) with selected ion monitoring of PGH₂-EA metabolite peaks at *m*/*z* = 418. Quantitation of COX-2 activity is accomplished by comparing the area of the PGH₂-EA metabolite peak to that of the internal standard.

### REFERENCES

Bisogno, T., Melck, D., De Petrocellis, L., & Di Marzo, V. (1999) J. Neurochem. 72:2113-2119.
DuBois, RN, Radhika, A, Reddy, BS, Entingh, AJ. (1996) Gastroenterology 110:1259.
Cohen, et al. U.S. Patent No. 5,589,575, *Purification of hapten-carrier generated antibodies.*
Cayman Chemical, 1180 E. Ellsworth Road, Ann Arbor, MI 48108; 800-364-9897; *Prostaglandin E₂ affinity purification kit,* Catalog No. 514018..
Devane WA, Hanus L, Breuer A, Pertwee RG, Stevenson LA, Griffin D, Mandelbaum A, Etinger A, & Mechoulam R (1992) Science 258:1946-1949.
Eberhart, CE, Coffey, RJ, Radhika, A, et al. (1994) Gastroenterology 107:1183.
Fosslien, E. (2000) Ann. Clin. Lab. Sci. 30:3-21.
Jeon, YJ, Yang, KH, Pulaski, JT, & Kaminski, NE. (1996) Mol. Pharmacol. 50:334-341.
Kalgutkar, AS, Kozak, KR, Crews, BC, Hochgesang, Jr. GP, & Marnett, LJ. (1998) J. Med. Chem. 41: 4800-4818.
Kalgutkar, AS, Crews, BC, Rowlinson, SW, Marnett, AB, Kozak, KR, Remmel, RP, & Marnett, LJ. (2000) Proc. Natl. Acad. Sci. USA 97: 925-930.
Kennedy, BP, Chan, C-C, Culp, SA, & Cromlish, WA. (1993) Biochem. Biophys. Res. Commun. 197:494-500.
Kozak, KR, Rowlinson, SW, & Marnett, LJ. (2000) J. Biol. Chem. 275:33744-33749.
Landino, LM, Crews, BC, Timmons, MD, Morrow, JD, & Marnett, LJ. (1996) Proc. Natl. Acid. Sci. USA 93:15069-15074.
MacPherson, JC, Pavlovich, JG, & Jacobs, RS. (1996) Biochim. Biophys. Acta 1303:127-136.
Marnett, LJ, Siedlik, PH, Ochs, RC, Pagels, WD, Das, M, Honn, KV., Warnock, RH, Tainer, BE, & Eling, TE. (1984) Mol. Pharmacol. 26:328-335.
Masferrer, JL, Zweifel, BS, Manning, PT, Hauser, SD, Leahy, KM., Smith, WG, Isakson, PC, & Seibert, K. (1994) Proc. Natl. Acid. Sci. USA 91, :28-3232.
McCafferty, J., Hoogenboom, H., & Chiswell, D. (1996) Antibody Engineering, a Practical Approach, IRL Press @ Oxford University Press.
Mernaugh, R. & Mernaugh, G. (1994) Methods for the Production of Monoclonal Antibodies, in Molecular Methods in Plant Pathology, Ed by RP Singh and US Singh, pg. 343-365.
Odenwaller, R, Chen, Y-NP., & Marnett, LJ. (1990) Methods. Enzymol. 187: 479-485.
Rowlinson, SW, Crews, BC., Lanzo, CA, & Marnett, LJ. (1999) J. Biol. Chem. 274: 23305-23310.
Rowlinson, SW, Crews, BC, Goodwin, DC, Schneider, C, Gierse, JK, & Marnett, L. J. (2000) J. Biol. Chem. 274:6586-6591
Sheehan, KM, Sheahan, K, O'Donoghue, DP, et al. (1999) JAMA 282:1254.
Sheng, H, Shao, J, Kirkland, SC, et al. (1997) J. Clin. Invest. 99:2254.
So, O-Y, Scarafia, LE, Mak, AY, Callan, OH, & Swinney, DC (1998) J. Biol. Chem. 273:5801-5807.
Stella, N., Schweitzer, P., & Piomelli, D. (1997) Nature 388:773-778.
Tsujii, M, Kawano, S, Du Bois, RN. (1997) Proc. Natl. Acad. Sci. USA 94:3336.
Vane, JR, Mitchell, JA, Appleton, I, Tomlinson, A, Bishop-Bailey, D, Croxtall, J, & Willoughby, DA (1994) Proc. Natl. Acad. Sci. USA 91:2046-2060.
Wadleigh, DJ, Reddy, ST, Kopp, E, Ghosh, S, & Herschman, HR (2000) J. Biol. Chem. 275:6259-6266.
Yu, M, Ives, D, & Ramesha CS (1997) J. Biol. Chem. 272:21181-21186.

## Claims

1. A method of measuring an activity of a COX-2 enzyme comprising:
a. quantifying, *in vitro,* an amount of a PGH₂-EA metabolite in a sample from a mammalian subject; and
b. relating the amount of the PGH₂-EA metabolite quantified in the sample to the activity of the COX-2 enzyme in the subject from which the sample was taken.

2. A method of detecting an activity of a COX-2 enzyme comprising:
a. detecting, *in vitro,* an amount of a PGH₂-EA metabolite in a sample from a mammalian subject; and
b. comparing the detected amount to at least one of a standard, standard curve, or a previously detected amount from the subject, wherein the amount of the PGH₂-EA metabolite in the sample indicates the activity of the COX-2 enzyme in the subject from which the sample was taken; and
c. relating the detected amount to a disease state or the progression of a disease state in the subject from which the sample was taken, wherein the disease state is cancer or a disease associated with inflammation.

3. A method of measuring an activity of a COX-2 enzyme comprising:
a. measuring, *in vitro,* an amount of a PGH₂-EA metabolite in a sample from a mammalian subject;
b. relating the amount measured in the sample of the mammalian subject to the activity of the COX-2 enzyme in the mammalian subject; and
c. associating the activity of the COX-2 enzyme to a disease state in the mammalian subject or the progression of a disease state in the mammalian subject from which the sample was taken by comparing the activity of the COX-2 enzyme in the subject with at least one of a standard, standard curve, or a previously measured COX-2 activity in the subject, wherein the disease state is cancer or a disease associated with inflammation.

4. The method of claim 2 or 3, wherein the disease state is associated with cancer, including the presence of or progression of a tumor, and/or the disease state is associated with inflammation, and/or the disease state is acute appendicitis, asthma, myocardial infarction, immunological disease processes, infection with viruses or bacteria, malignancy and metastasis, endotoxemia and reperfusion injury, cell-proliferative diseases, psoriasis, pemphigus vulgaris, Behcet's syndrome, acute respiratory distress syndrome, ischemic heart disease, post-dialysis syndrome, leukemia, acquired immune deficiency syndrome, septic shock, acute inflammation, lipid histiocytosis.

5. A method for screening for the presence of a tumor in a subject comprising measuring an amount of PGH₂-EA metabolite in a sample from said subject and relating the amount of PGH₂-EA metabolite measured to the existence of a tumor.

6. A method of monitoring the effectiveness of anti-cancer or inflammation treatment in a subject comprising:
a. measuring a first amount of PGH₂-EA metabolite in a first sample from said subject taken prior to said treatment,
b. measuring a second amount of PGH₂-EA metabolite in a second sample from said subject, wherein any change in the second amount of PGH₂-EA metabolite is indicative of the effectiveness of said treatment.

7. The method of any of the above claims, wherein the PGH₂- EA metabolite is selected from the group consisting of PGB₂-EA, PGD₂-EA, PGE₂-EA, PGF₂α-EA, TxB₂-EA, 6-keto-PGF₁α-EA, 15-keto-PGE₂-EA, 13,14-dihydro-15-keto-PGE₂-EA, PGG₂-EA, PGH₂-EA, PGA₂-EA, PGJ₂-EA, PGJ₂-EA derivatives, bicyclo-PGE₂-EA, 6-keto-PGF₁α-EA, TxA₂-EA and PGI₂-EA.

8. The method of any of the above claims, wherein the sample is urine, blood, plasma, cerebrospinal fluid, saliva, sputum, bile, joint fluid, biopsy, and/or conditioned media from a cell culture.

9. The method of claim 2, wherein the detecting step further comprises generating a mass chromatogram of the PGH₂-EA metabolites; and/or wherein the detecting step includes an immunoassay step.

## Patentansprüche

1. Verfahren zum Messen einer Aktivität von einem COX-2-Enzym, das Folgendes umfasst:
a. Quantifizieren, *in vitro*, einer Menge von einem PGH₂-EA-Metaboliten in einer Probe von einem Säugerindividuum; und
b. Beziehen der Menge des in der Probe quantifizierten PGH₂-EA-Metaboliten auf die Aktivität des COX-2-Enzyms in dem Individuum, dem die Probe entnommen wurde.

2. Verfahren zum Nachweis einer Aktivität von einem COX-2-Enzym, das Folgendes umfasst:
a. Nachweis, *in vitro,* einer Menge von einem PGH₂-EA-Metaboliten in einer Probe von einem Säugerindividuum; und
b. Vergleich der nachgewiesenen Menge mit mindestens einer von einem Standard, einer Standardkurve oder einer zuvor nachgewiesenen Menge von dem Individuum, worin die Menge des PGH₂-EA-Metaboliten in der Probe die Aktivität des COX-2-Enzyms in dem Individuum anzeigt, dem die Probe entnommen wurde; und
c. Beziehen der nachgewiesenen Menge auf einen Krankheitszustand oder auf die Progression eines Krankheitszustands in dem Individuum, dem die Probe entnommen wurde, worin der Krankheitszustand Krebs oder eine mit einer Entzündung assoziierte Erkrankung darstellt.

3. Verfahren zum Messen einer Aktivität von einem COX-2-Enzym, das Folgendes umfasst:
a. Messen, *in vitro,* einer Menge von einem PGH₂-EA-Metaboliten in einer Probe von einem Säugerindividuum;
b. Beziehen der in der Probe des Säugerindividuums gemessenen Menge auf die Aktivität des COX-2-Enzyms in dem Säugerindividuum; und
c. Assoziieren der Aktivität des COX-2-Enzyms mit einem Krankheitszustand in dem Säugerindividuum oder mit der Progression eines Krankheitszustands in dem Säugerindividuum, dem die Probe entnommen wurde, durch Vergleich der Aktivität des COX-2-Enzyms in dem Individuum mit mindestens einer von einem Standard, einer Standardkurve oder einer zuvor gemessenen COX-2-Aktivität in dem Individuum, worin der Krankheitszustand Krebs oder eine mit einer Entzündung assoziierte Erkrankung darstellt.

4. Verfahren nach Anspruch 2 oder 3, worin der Krankheitszustand mit Krebs, einschließlich der Gegenwart oder der Progression eines Tumors assoziiert ist, und/oder der Krankheitszustand mit einer Entzündung assoziiert ist, und/oder der Krankheitszustand Folgendes darstellt: akute Appendizitis, Asthma, einen Myokardinfarkt, immunologische Krankheitsprozesse, eine Infektion mit Viren oder Bakterien, eine Malignität und Metastasen, eine Endotoxämie und Reperfusionsverletzung, zellproliferative Erkrankungen, Psoriasis, Pemphigus vulgaris, das Behçet-Syndrom, das akute respiratorische Distresssyndrom, eine ischämische Herzkrankheit, das Postdialysesyndrom, Leukämie, das erworbene Immundefizienzsyndrom, septischen Schock, eine akute Entzündung und Lipid-Histiozytose.

5. Verfahren zum Screening auf das Vorliegen eines Tumors in einem Individuum, umfassend das Messen einer Menge des PGH₂-EA-Metaboliten in einer Probe von einem Individuum und Beziehen der Menge des gemessenen PGH₂-EA-Metaboliten auf das Vorliegen eines Tumors.

6. Verfahren zum Überwachen der Wirksamkeit der Antikrebs- oder Entzündungsbehandlung bei einem Individuum, das Folgendes umfasst:
a. Messen einer ersten Menge des PGH₂-EA-Metaboliten in einer ersten Probe von dem Individuum, die vor der Behandlung entnommen wurde,
b. Messen einer zweiten Menge des PGH₂-EA-Metaboliten in einer zweiten Probe von dem Individuum, worin jedwede Änderung der zweiten Menge des PGH₂-EA-Metaboliten für die Wirksamkeit der Behandlung indikativ ist.

7. Verfahren nach einem der vorstehenden Ansprüche, worin der PGH₂-EA-Metabolit aus der Gruppe ausgewählt ist, bestehend aus : PGB₂-EA, PGD₂-EA, PGE₂-EA, PGF₂α-EA, TxB₂-EA, 6-Keto-PGF₁α-EA, 15-Keto-PGE₂-EA, 13,14-Dihydro-15-keio-PGE₂-EA, PGG₂-EA, PGH₂-EA, PGA₂-EA, PGJ₂-EA, PGJ₂-EA-Derivaten, Bicyclo-PGE₂-EA, 6-Keto-PGF₁α-EA, TxA₂-EA und PGI₂-EA.

8. Verfahren nach einem der vorstehenden Ansprüche, worin die Probe Urin, Blut, Plasma, Cerebrospinalflüssigkeit, Speichel, Sputum, Galle, Gelenkflüssigkeit, eine Biopsie und/oder ein konditioniertes Medium aus einer Zellkultur darstellt.

9. Verfahren nach Anspruch 2, worin der Nachweisschritt ferner die Herstellung eines Massenchromatogramms von den PGH₂-EA-Metaboliten umfasst; und/oder worin der Nachweisschritt einen Immunassay-Schritt einschließt.

## Revendications

1. Méthode de mesure d'une activité enzymatique COX-2, qui comprend :
a. la quantification, *in vitro,* d'une quantité d'un métabolite de type PGH₂-EA dans un échantillon obtenu chez un sujet mammifère ; et
b. la corrélation entre la quantité du métabolite de type PGH₂-EA mesurée dans l'échantillon et l'activité enzymatique COX-2 chez le sujet chez lequel l'échantillon a été obtenu.

2. Méthode de détection d'une activité enzymatique COX-2, qui comprend :
a. la détection, *in vitro,* d'une quantité d'un métabolite de type PGH₂-EA dans un échantillon obtenu chez un sujet mammifère ; et
b. la comparaison entre la quantité détectée et au moins un étalon de référence, une courbe d'étalonnage ou une quantité détectée antérieurement chez le sujet, où la quantité du métabolite de type PGH₂-EA présente dans l'échantillon est indicative de l'activité enzymatique COX-2 chez le sujet chez lequel l'échantillon a été obtenu ; et
c. la corrélation entre la quantité détectée et un état pathologique ou l'évolution d'un état pathologique chez le sujet chez lequel l'échantillon a été obtenu, où l'état pathologique est un cancer ou une affection associée à une inflammation.

3. Méthode de mesure d'une activité enzymatique COX-2, qui comprend :
a. la mesure, *in vitro,* d'une quantité d'un métabolite de type PGH₂-EA dans un échantillon obtenu chez un sujet mammifère ; et
b. la corrélation entre la quantité du métabolite COX-2 mesurée dans l'échantillon du sujet mammifère et l'activité enzymatique COX-2 chez le sujet mammifère ; et
c. l'association entre l'activité enzymatique COX-2 et un état pathologique ou l'évolution d' un état pathologique chez le sujet mammifère chez lequel l'échantillon a été obtenu par une comparaison entre l'activité enzymatique COX-2 chez le sujet et au moins un étalon de référence, une courbe d' étalonnage ou une activité COX-2 mesurée antérieurement chez le sujet, où l'état pathologique est un cancer ou une affection associée à une inflammation.

4. Méthode de la revendication 2 ou 3, où l'état pathologique est associé à un cancer, y compris à la présence ou à l'évolution d'une tumeur, et/ou l'état pathologique est associé à une inflammation, et/ou l'état pathologique est une appendicite aiguë, l'asthme, un infarctus du myocarde, des processus pathologiques immunologiques, une infection par des virus ou bactéries, une affection maligne et métastatique, une endotoxémie et une lésion de reperfusion, des maladies faisant intervenir une prolifération cellulaire, un psoriasis, un pemphigus vulgaire, un syndrome de Behçet, un syndrome de détresse respiratoire aiguë, une cardiopathie ischémique, un syndrome post-dialytique, une leucémie, un syndrome d'immunodéficience acquise, un choc septique, une inflammation aiguë ou une histiocytose lipidique.

5. Méthode de dépistage de la présence d'une tumeur chez un sujet, qui comprend la mesure d'une quantité d'un métabolite de type PGH₂-EA dans un échantillon obtenu chez ledit sujet et la corrélation entre la quantité de métabolite de type PGH₂-EA mesurée et l'existence d'une tumeur.

6. Méthode de monitorage de l'efficacité d'un traitement anticancéreux ou d'un traitement anti-inflammatoire chez un sujet, qui comprend :
a. la mesure d'une première quantité d'un métabolite de type PGH₂-EA dans un premier échantillon obtenu chez ledit sujet avant l'introduction du dit traitement ;
b. la mesure d'une seconde quantité d'un métabolite de type PGH₂-EA dans un second échantillon obtenu chez ledit sujet, où aucun changement dans la seconde quantité d'un métabolite de type PGH₂-EA est indicatif de l'efficacité du dit traitement.

7. Méthode de l'une quelconque des revendications précédentes, où le métabolite de type PGH₂-EA est sélectionné dans le groupe consistant en des dérivés PGB₂-EA, PGD₂-EA, PGE₂-EA, PGF₂α-EA, TxB₂-EA, 6-céto-PGF₁α-EA, 15-céto-PGE₂-EA, 13,14-dihydro-15-céto-PGE₂-EA, PGG₂-EA, PGH₂-EA, PGA₂-EA, PGJ₂-EA et PGJ₂-EA, le bicyclo-PGE₂-EA, la 6-céto-PGF₁α-EA, le TxA₂-EA et la PGI₂-EA.

8. Méthode de l'une quelconque des revendications précédentes, où l'échantillon est un échantillon d'urine, de sang, de plasma, de liquide céphalorachidien, de salive, d'expectorations, de bile ou de liquide articulaire, une biopsie et/ou des milieux conditionnés obtenus à partir d'une culture cellulaire.

9. Méthode de la revendication 2, où l'étape de détection comprend également l'enregistrement d'un chromatogramme de masse des métabolites de type PGH₂-EA ; et/ou où l'étape de détection comprend une étape de immunodosage.
